# EUROPÄISCHE PATENTSCHRIFT

(11) **EP 1 274 994 B1**
(45) Veröffentlichungstag und Bekanntmachung des Hinweises auf die Patenterteilung: **08.11.2006**
(21) Anmeldenummer: 01936176.5
(22) Anmeldetag: 03.04.2001
(51) Int. Cl.: G01N 31/22, A61B 5/00, A61B 5/103

(54) **VERFAHREN ZUR HERSTELLUNG EINES PFLASTERS FÜR DIE BESTIMMUNG EINER ABNORMALEN HAUTTROCKENHEIT**
METHOD OF PREPARATION OF A BAND AID FOR DETERMINING AN ABNORMAL SKIN DRYNESS
PROCEDE DE PREPARATION D'UN PANSEMENT POUR LA DETERMINATION D'UNE SECHERESSE ANORMALE DE LA PEAU

(30) Priorität: 15.04.2000 DE 10018790
(43) Veröffentlichungstag der Anmeldung: 15.01.2003
(73) Patentinhaber: Cremerius, Alois, 40477 Düsseldorf (DE); Thissen, Rudolf, 41849 Wassenberg (DE); Zick, Gisela, 49809 Lingen/Ems (DE)
(72) Erfinder: Cremerius, Alois, 40477 Düsseldorf (DE); Thissen, Rudolf, 41849 Wassenberg (DE); Zick, Gisela, 49809 Lingen/Ems (DE)
(74) Vertreter: Paul, Dieter-Alfred
(86) Internationale Anmeldenummer: PCT/EP2001/003776
(87) Internationale Veröffentlichungsnummer: WO 2001/079836

(56) Entgegenhaltungen:
- EP-A- 0 430 608
- WO-A-93/14699
- YAMAMURA T ET AL: "SIMPLE MONOCHROMATIC REFRACTOMETER FOR TRANSEPIDERMAL WATER LOSS TEWL" JOURNAL OF DERMATOLOGICAL SCIENCE, Bd. 1, Nr. 3, 1990, Seiten 201-206, XP001019738 ISSN: 0923-1811

## Beschreibung

Die vorliegende Erfindung betrifft ein Verfahren zur Herstellung eines Pflasters für die Verwendung zur Bestimmung einer abnormalen Hauttrockenheit und insbesondere zur Diagnostik des diabetischen Fußsyndroms.

Im Rahmen der Behandlung von Diabetes nehmen die Früherkennung von Folgeerkrankungen wie beispielsweise die Diagnostik des "diabetischen Fußsyndroms" (DFS) und die Poly-neuropathie-Diagnostik (PNP) einen wichtigen Stellenwert ein. Allein in der Bundesrepublik Deutschland gibt es etwa 5 Millionen Diabetiker, die alle durch den diabetischen Fuß, welche die häufigste Folgeerkrankung der Stoffwechselstörung darstellt, bedroht sind. Störungen bestimmter Nervenfunktionen in Kombination mit Durchblutungsstörungen führen zu Hautveränderungen, und diese wiederum zu praktisch unbeeinflußbaren tiefen Gewebsdefekten mit nachfolgenden Infektionen. Häufig müssen die betroffenen Gliedmaßen amputiert werden. Allein in Deutschland beträgt die durch das diabetische Fußsyndrom verursachte Anzahl von Amputationen an der unteren Extremität jährlich ca. 28.000 bis 30.000. Trotz unterschiedlichster Bemühungen konnte diese Zahl in den vergangenen Jahren nicht reduziert werden, ganz im Gegenteil wächst die Amputationsrate jährlich um ca. 2 %. Hier kann nur eine praktikable Früherkennungsmethode mit einer darauf basierenden, ebenso praktikablen Hautpflegeanleitung Abhilfe schaffen. Das erste diagnostisch zugängliche und relativ einfach therapeutisch zu beeiflussende Frühsymptom des diabetischen Fußes ist eine abnorme Hauttrockenheit, welche die Haut des Fußes unelastisch und damit mechanisch verletzbar macht. Kleinste Bagatellverletzungen und die zwangsläufige Besiedelung mit ubiquitären Krankheitserregern sind dann der Beginn des diabetischen Fußsyndroms. Entsprechend sind zur Frühdiagnostik des diabetischen Fußes unterschiedliche Geräte entwickelt worden, um die Hauttrockenheit zu erfassen. Aus der DE 198 33 440 A1 ist beispielsweise eine Vorrichtung bekannt, welche die Hauttrockenheit über den elektrischen widerstand bzw. den elektrischen Leitwert erfaßt. Diese Geräte sind mit einem entsprechenden apparativen Aufwand verbunden.

Das Dokument EP-A-0 340 608 beschreibt einen medizinischen Verband mit einer die Haut oder Wunde bedeckenden adhäsiven Schicht, in der wasserlösliche oder quellfähige Kolloide in einer elastischen Substanz dispergiert sind. Zusätzlich dazu sind in diesem Verband Indikatormittel in Form von temperatursensiblen Flüssigkristallen vorgesehen, die es ermöglichen sollen, die Temperaturänderungen in einer wunde bzw. auf der Haut beobachten zu können, ohne den Verband abnehmen zu müssen. Um durch einen Farbumschlag anzeigen zu können, daß eine patogene Wunde näßt und der Verband gegebenenfalls gewechselt werden muß, wird u.a. der Einsatz von Kobalt-Chlorid vorgeschlagen.

Eine weitere Möglichkeit des Einsatzes von Kobalt-Chlorid schlägt die Veröffentlichung Yamamura, T. et al: Simple monochromatic refractometer for transepidermal water loss (TEWL)" Journal of Dermatologicla Science, Band 1, Nr. 3, 1990, Seiten 201 - 206 vor, in welchem ein Verfahren zur Untersuchung pathogener vermehrter Schweißsekretion aufgrund fehlerhafter Wasserspeicherfunktion der Epidermis beschrieben wird.

Hierzu wird ein mit Kobalt-Chlorid getränktes und getrocknetes Filterpapier auf die Hautpartie eines Patienten aufgetragen und mit einem Klebestreifen fixiert. Das Filterpapier nimmt die auf der Hautoberfläche entwickelte Feuchtigkeit auf. Bei Aufnahme einer bestimmten Feuchtigkeitsmenge erfährt das ursprünglich blau gefärbte Filterpapier einen Farbumschlag nach rot. Nach einer vorher festgelegten Zeit wird das Filterpapier von der Hautpartie gelöst und in eine Auswerteeinrichtung eingesetzt, in der es mit einem monochromatischen Licht mit einer Wellenlänge von 670 nm angestrahlt wird. Ein Teil des Lichtes wird von der Oberfläche des mit Kobalt-Chlorid getränkten Filterpapiers in Abhängigkeit des Verfärbungsgrades reflektiert. Diese Reflexion wird über einen Phototransistor ausgewertet. Der Grad der Farbänderung von blau nach rot entspricht dann einer bestimmten Menge absorbierten Wassers.

Weder diese Vorrichtung noch die vorausgegangene sind dazu geeignet, eine eventuell pathogene Hauttrockenheit zu detektieren.

Aufgabe der Erfindung ist es daher, ein Verfahren zur Herstellung eines Pflasters anzugeben, mit welchem sich auf einfache weise feststellen läßt, ob der Feuchtigkeitsgehalt einer Hautpartie, insbesondere der Fußsohlen, noch im normalen Bereich befindet oder nicht mehr.

Diese Aufgabe ist erfindungsgemäß durch dass Verfahren des Anspruchs 1 gelöst.

Der Erfindung liegt damit der Gedanke zugrunde, den Flüssigkeitsanteil des Schweißes auf eine Hautpartie, beispielsweise der Fußsohle, semiquantitativ in einem reproduzierbaren, standartisierten und wenig fehleranfälligen sowie einfach zu handhabenden Test zu bestimmen, indem ein Reaktionsträger auf die jeweilige Hautpartie aufgebracht wird, der mit Kobalt-(II)-Chlorid einen Indikator aufweist, der im trockenen zustand eine Blaufärbung aufweist, die bei einem gewissen Feuchtigkeitsgehalt, welcher einen normalen Hautfeuchte entspricht, in eine Rotfärbung umschlägt. Anhand der Farbe, die der Reaktionsträger aufweist, nachdem er einige Minuten mit der Haut in Berührung gestanden hat, läßt sich somit einwandfrei feststellen, ob eine abnorme Hauttrockenheit vorliegt oder nicht.

Durchgeführt werden kann dieses Verfahren zur Indikation von abnormaler Hauttrockenheit mittels eines entsprechenden Indikatorpflasters, welches einen flächigen Grundkörper aus einem flexiblen Material, der auf seiner einen Seite mit einem Haftmaterial versehen ist und auf dieser Seite den Reaktionsträger hält, aufweist. Dieses Indikatorpflaster ist mit einer luft- und feuchtigkeitsundurchlässigen Schutzschicht oder Schutzhülle umgeben, die erst kurz vor der Benutzung entfernt wird, um eine frühzeitige Reaktion des Kobalt-(II)-Chlorids zu verhindern.

Hinsichtlich weiterer vorteilhafter Ausgestaltungen der Erfindung wird auf die Unteransprüche sowie die nachfolgende Beschreibung eines Ausführungsbeispiels unter Bezugnahme auf die beiliegende Zeichnung verwiesen. In der Zeichnung zeigt:

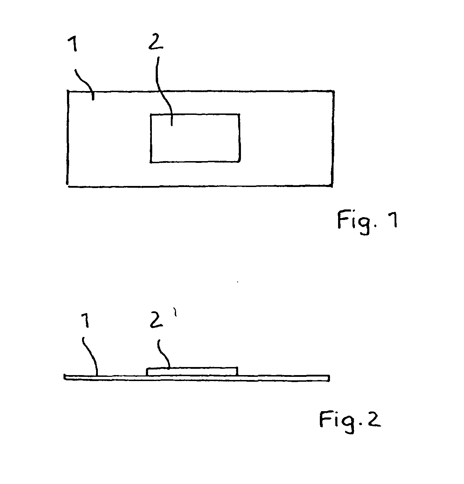

- Fig. 1: ein Indikatorpflaster gemäß der vorliegenden Erfindung in Draufsicht und
- Fig. 2: das Indikatorpflaster aus Fig. 1 in schematischer Seitenansicht.

In der Zeichnung ist ein Indikatorpflaster gemäß der vorliegenden Erfindung dargestellt. Das Indikatorpflaster besitzt einen flächigen Grundkörper 1 aus einem flexiblen Material, hier einer transparenten und einseitig mit einer Haftschicht versehenen Klebefolie. Andere übliche Pflastermaterialien können auch verwendet werden. An der die Haftschicht aufweisenden hautzugewandten Seite der Klebefolie 1 ist ein textiler Reaktionsträger 2 z.B aus Zellstoff oder Vlies angebracht, der mit einer 6,25 %igen Kobalt-(II)-Chloridlösung imprägniert ist. Bei der Herstellung wird die Kobalt-(II)-Chloridlösung auf den Reaktionsträger 2 aufgebracht und bis zur Blauverfärbung, beispielsweise durch Erwärmung auf etwa 40°C, getrocknet, bevor der Reaktionsträger 2 auf den Pflasterstreifen 1 aufgeklebt wird. In der Zeichnung gut erkennbar ist, daß der Reaktionsträger 2 kleiner als die Klebefolie 1 und etwa mittig auf dieser angebracht ist, so daß um den Reaktionsträger 2 herum ein Kleberand entsteht, wie dies von herkömmlichen Pflastern her bekannt ist.

Nicht dargestellt ist, daß das Pflaster von einer Schutzhülle aus einem feuchtigkeitsundurchlässigen Material umgeben ist, welche verhindert, daß das Kobalt-(II)-Chlorid mit Feuchtigkeit in Berührung kommt.

Zur Früherkennung einer abnormalen Hauttrockenheit wird das Pflaster aus der Schutzhülle entnommen und auf eine zu testende Hautpartie geklebt. Sofern die Haut eine normale Feuchtigkeit besitzt, schlägt die Blaufärbung des Kobalt-(II)-Chlorids durch den Kontakt mit dem Schweiß der Haut in eine Rotfärbung um. Sofern eine zu geringe Hautfeuchtigkeit - mit andern Worten eine abnormale Hauttrockenheit - vorliegt, bleibt es bei der Blauverfärbung. Auf diese Weise läßt sich mit einfachen Mitteln zuverlässig semiquantitativ feststellen, ab eine abnormale Hauttrockenheit vorliegt oder nicht. Tests haben gezeigt, daß bei gesunder Haut ein Farbumschlag von blau über weiß und rosa nach rot innerhalb von vier Minuten stattfindet, während eine pathologische Hautsekretion mit Sicherheit vorliegt, wenn ein solcher Farbumschlag mach 20 Minuten noch nicht stattgefunden hat.

Das erfindungsgemäße Verfahren ist zur Diagnostik in allen Bereichen geeignet, in denen eine krankhaft verminderte Schweißsekretion eine Rolle spielt. Insbesondere kann es im neurologischen Bereich anstelle der Minor- und Ninhydrin-Tests zum Nachweis einer pathologischen Schweißsekretion eingesetzt werden.

## Patentansprüche

1. Verfahren zur Herstellung eines Pflasters für die Verwendung zur Bestimmung einer abnormalen Hauttrokkenheit, bei dem ein Reaktionsträger (2) mit einer 3,15 bis 25 prozentigen Kobalt-(II)-Chloridlösung imprägniert und anschließend bis zur Blaufärbung des Kobalt-(II)-Chlorids getrocknet wird,
der Reaktionsträger (2) an einer Seite eines flächigen Grundkörpers (1) aus einem flexiblen Material angebracht wird, und
der Reaktionsträger (2) mit einem luft- und feuchtigkeitsundurchlässigen Schutzmaterial (3) abgedeckt wird.

2. Verfahren nach Anspruch 1, **dadurch gekennzeichnet, daß** der Reaktionsträger (2) mit einer 6,25 prozentigen Kobalt-(II)-Chloridlösung imprägniert wird.

3. Verfahren nach Anspruch 1 oder 2, **dadurch gekennzeichnet, daß** der Reaktionsträger (2) aus zellstoff besteht.

4. Verfahren nach Anspruch 1 oder 2, **dadurch gekennzeichnet, daß** ein Reaktionsträger (2) aus einem vliesmaterial verwendet wird.

5. Verfahren nach einem der vorherigen Ansprüche, **dadurch gekennzeichnet, daß** der Reaktionsträger (2) mit einer luft- und feuchtigkeitsundurchlässigen Schutzfolie (3) abgedeckt wird.

6. Verfahren nach einem der vorherigen Ansprüche, **dadurch gekennzeichnet, daß** als Grundkörper (1) eine einseitig klebende Folie verwendet wird.

7. Verfahren nach Anspruch 6, **dadurch gekennzeichnet, daß** der Reaktionsträger (2) kleiner als der Grundkörper (1) und insbesondere mittig auf diesem aufgebracht ist.

## Claims

1. A method for producing a plaster for use in the determination of abnormal skin dryness, in which
a reaction support (2) is impregnated with a 3.15- to 25-weight percent cobalt(II) chloride solution and then dried until the cobalt(II) chloride has a blue color;
the reaction support (2) is applied onto one side of a planar base element (1) made of a flexible material;
the reaction support (2) is covered with a protective material (3) that is impermeable to air and moisture.

2. The method as defined in Claim 1, wherein the reaction support (2) is impregnated with a 6.25-weight percent cobalt(II) chloride solution.

3. The method as defined in Claim 1 or 2, wherein the reaction support (2) is made of cellulose.

4. The method as defined in Claim 1 or 2, wherein a reaction support (2) made of a nonwoven material is used.

5. The method as defined in one of the foregoing claims, wherein the reaction support (2) is covered with a protective film (3) that is impermeable to air and moisture.

6. The method as defined in one of the foregoing claims, wherein a film that is adhesive on one side is used as the base element (1).

7. The method as defined in Claim 6, wherein the reaction support (2) is smaller than the base element (1) and is applied, in particular, centeredly thereon.

## Revendications

1. Procédé pour la fabrication d'un emplâtre utilisé dans la déte r-mination d'une sécheresse anormale de la peau, où un support réactif (2) est imprégné avec une solution de chlorure de cobalt (II) avec la concentration de 3,15 jusqu'à 25% et en continuation séché jusqu'à la coloration en bleu du chlorure de cobalt (II),
- où le support réactif (2) est fixé sur un côté d'un corps de b ase (1) étendu d'un matériel flexible, et
- le support réactif (2) est couvert avec un matériel de protect ion (3) imperméable pour l'eau et l'humidité.

2. Procédé selon la revendication 1, **caractérisé en ce que**, le su p-port réactif (2) est imprégné avec une solution de 6,25 % chlorure de cobalt (II).

3. Procédé selon la revendication 1 ou 2, **caractérisé en ce que**, le support réactif (2) se compose de cellulose.

4. Procédé selon la revendication 1 ou 2, **caractérisé en ce que**, s'utilise un support réactif (2) d'un matériel de couverture.

5. Procédé selon l'une des revendications antérieures, **caractérisé en ce que**, le support réactif (2) est couvert avec une feuillé de protection (3) imperméable pour l'air et l'humidité.

6. Procédé selon l'une des revendications antérieures, **caractérisé en ce qu'**on utilise comme corps de base (1) une feuille adhésive sur un côté.

7. Procédé selon la revendication 6, **caractérisé en ce que**, le su p-port réactif (2) est plus petit que le corps de base (1) et spécial e-ment fixé sur celui-ci.
